# EUROPEAN PATENT APPLICATION

(11) **EP 4 670 743 A1**
(43) Date of publication of application: **31.12.2025**
(21) Application number: 24760678.3
(22) Date of filing: 26.02.2024
(51) Int. Cl.: A61K 47/69, A61K 47/64, A61K 31/352, A61P 29/00, A61P 21/00

(54) **ALBUMIN NANOCOMPOSITE COMPRISING PHYTOCHEMICAL AND COMPOSITION FOR IMPROVING MUSCLE DISEASE CONTAINING SAME**

(30) Priority: 24.02.2023 KR 20230024988; 23.02.2024 KR 20240026544
(71) Applicant: Seoul National University R&DB Foundation, Seoul 08826 (KR)
(72) Inventor: LEE, Yun Sang, Gyeonggi-do 16851 (KR); LEE, Ki Won, Seoul 08826 (KR); JANG, Young Jin, Seoul 02028 (KR); LEE, Jaesuk, Seoul 05280 (KR); AHN, Byeong Min, Gyeonggi-do 11812 (KR); PARK, Ji Yong, Incheon 22002 (KR); YOO, Ran Ji, Seoul 01727 (KR); AHN, Youngchan, Seoul 03123 (KR); KIM, Kyuwan, Seoul 02057 (KR); KIM, Jin Sil, Seoul 02863 (KR); AHN, Heeju, Seoul 01848 (KR)
(74) Representative: AWA Sweden AB
(86) International application number: PCT/KR2024/095458
(87) International publication number: WO 2024/177490

(57) **Abstract**

The present disclosure relates to an albumin nanocomposite comprising a phytochemical and a composition for muscle disease comprising the same, and more specifically, to an albumin nanocomposite comprising a phytochemical that exhibits an effect of inhibiting muscle loss caused by oxidative stress or inflammatory response and promoting the differentiation of myoblasts into muscle cells. The albumin nanocomposite comprising the phytochemical effectively delivers the phytochemical by targeting immune cells that induce reactive oxygen species and inflammatory responses. Accordingly, by regulating muscle-loss signaling pathways induced by reactive oxygen species and inflammatory responses, the nanocomposite inhibits muscle cell atrophy and promotes differentiation. Therefore, a composition comprising the albumin nanocomposite comprising the phytochemical according to the present disclosure can be provided as a composition for improving, preventing, or treating muscle diseases.

## Description

### [Technical Field]

The present invention relates to an albumin nanocomposite comprising a phytochemical, and a composition for improving muscle disease comprising the same, and more specifically, to an albumin nanocomposite comprising a phytochemical for preventing or improving muscle disease by inhibiting muscle loss caused by oxidative stress or inflammatory response and promoting the differentiation of myoblasts into muscle cells, and a composition for improving muscle disease comprising the same.

### [Background Art]

In recent years, as the elderly population is rapidly increasing worldwide, the United Nations has predicted that the number of people aged 60 years or older will exceed 2 billion by 2050. With this rapid increase in the elderly population aged 65 and older, the quality of life of the elderly in an aging society has emerged as an important issue, and accordingly, the demand for the extension of healthy life expectancy is increasing.

Skeletal muscle is the largest organ in the human body, accounting for 40 to 50% of total body weight, and is responsible for various bodily functions, including maintaining body structure, physical activities such as walking, as well as involvement in energy homeostasis, thermogenesis, and glucose and amino acid metabolism. Sarcopenia is accompanied by a decrease in skeletal muscle mass and a decline in muscle function, and is one of the representative symptoms of aging. In 2017, as the World Health Organization (WHO) assigned a disease code to sarcopenia, experts have anticipated that many products aimed at preventing or improving this condition would be developed by food and pharmaceutical companies.

Such muscle loss occurs as a result of an imbalance between the synthesis and degradation of muscle proteins, increased cell death of muscle cells, and reduced regenerative capacity of muscles. Muscle loss and reduced strength are major physiological changes associated with aging and are known to proceed progressively from the age of 40. This reduction in muscle mass is accompanied by a general decline in physical function, which lowers the quality of life of individuals. It has been reported that elderly people with low muscle mass have higher mortality rates, and that sarcopenic individuals have significantly higher mortality even from the same disease. Known causes of sarcopenia include deficiencies in hormones such as growth hormones and sex hormones, imbalance between synthesis and degradation of muscle proteins, inactivity, obesity, increase in inflammatory cytokines, decline in mitochondrial function, and insulin resistance. In particular, it has been reported that with increasing age, obesity and visceral fat increase, which in turn elevates inflammatory cytokines and induces sarcopenia.

Currently, hormone therapy is commonly used for treating sarcopenia, however, it has been found to be ineffective, and various side effects are being continuously reported. Although many pharmaceutical companies have attempted clinical trials using inhibitors that suppress or block myostatin, all such trials have failed, highlighting the urgent need for new alternatives.

Since sarcopenia is caused by multiple factors, it has been reported that controlling a single pathway is insufficient to manage sarcopenia. Accordingly, in order to increase clinical success rates, the development of a technology capable of simultaneously controlling multiple pathways that cause muscle weakness to alleviate or treat sarcopenia is being demanded.

To develop such new materials, Korean Patent No. 10-2216599 discloses a health functional food material for improving sarcopenia, prepared by extracting active ingredients from Semisulcospira libertina and green tea. Korean Patent No. 10-2606636 discloses that Bacillus velezensis or a culture thereof has a preventive or ameliorative effect on sarcopenia and provides a composition for improving sarcopenia using the same.

However, none of the above-mentioned documents disclose a composition capable of selectively delivering a phytochemical to a tissue in which muscle loss has been induced, thereby enhancing the effect of improving muscle disease.

### [Disclosure]

### [Technical Problem]

An object of the present invention is to provide an albumin nanocomposite comprising a phytochemical.

Another object of the present invention is to provide a pharmaceutical composition for preventing or treating a muscle disease, comprising the albumin nanocomposite.

Still another object of the present invention is to provide a food composition for preventing or improving a muscle disease, comprising the albumin nanocomposite.

### [Technical Solution]

The present disclosure provides an albumin nanocomposite obtained by a click chemistry reaction between albumin conjugated with an azide (N₃) or cyclooctyne functional group and a transmitter conjugated with an azide (N₃) or cyclooctyne functional group, wherein a phytochemical is loaded on the surface of the albumin, the transmitter comprises a glucosyl group, and when the albumin is conjugated with the azide functional group, the transmitter is conjugated with the cyclooctyne functional group, and when the albumin is conjugated with the cyclooctyne functional group, the transmitter is conjugated with the azide functional group.

In the present disclosure, the phytochemical may be at least one selected from the group consisting of a phenolic compound and a triterpene.

In the present disclosure, the phenolic compound may be at least one selected from the group consisting of apigenin, citrusinol, flavone, flavonol, flavanone, flavanol, isoflavone, anthocyanin, stilbenoid, caffeic acid, p-coumaric acid, chrysin, tectochrysin, primetin, acacetin, luteolin, tangeritin, quercetin, kaempferol, genistein, daidzein, naringenin, hesperetin, cyanidin, catechin, curcumin, epigallocatechin gallate and resveratrol.

In the present disclosure, the triterpene may be at least one selected from the group consisting of ursolic acid, soyasapogenol A, soyasapogenol B, ursane, oleanane, lupane, saponin, sterol, oleanolic acid, lupeol, corosolic acid, maslinic acid, betulinic acid, ginsenoside, beta-sitosterol, campesterol, stigmasterol, lanosterol and germanicol.

In the present disclosure, the azide or cyclooctyne functional group introduced into the albumin may range from 1 to 14.

In the present disclosure, the nanocomposite may comprise 4 to 8 glucosyl groups.

In the present disclosure, the phytochemical may be loaded in an amount of 1 to 20 molecules.

In the present disclosure, the azide or cyclooctyne functional group may be conjugated to the 6th carbon of the glucosyl group.

In the present disclosure, the transmitter may further comprise a radioactive isotope, and the radioactive isotope may be at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹Fe, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn and ¹⁷⁷Lu.

In the present disclosure, the radioactive isotope may be labeled by a chelating agent, and the chelating agent may be at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihyroxy-7,10,18,21-tetraoxo-27-[N-acetylhydroxylamino]-6,11,17,22-tetraazaheptaeicosane]thiourea), DTPA (diethylenetriaminepentaacetic acid), N2S2 (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihyroxy-7,10,18,21-tetraoxo-27-[N-acetylhydroxylamino]-6,11,17,22-tetraazaheptaeicosane]thiourea) and HYNIC (hydrazinonicotinic acid).

In the present disclosure, the transmitter may further comprise a fluorescent material, and the fluorescent material may be at least one selected from the group consisting of FNR (Ferrodoxin NADP(+) reductase), cyanine-based fluorescent material, TAMRA (tetramethylrhodamine-5-maleimide), Flamma^{®} fluorescent material and ICG (indocyanine green).

In the present disclosure, the nanocomposite may selectively target a macrophage with overexpressed GLUT (Glucose Transporter) to deliver the phytochemical.

The present disclosure also provides a pharmaceutical composition for preventing or treating a muscle disease, comprising the albumin nanocomposite.

In the present disclosure, the muscle disease may be induced by an anticancer agent or an inflammatory response.

In the present disclosure, the anticancer agent may be at least one selected from the group consisting of etoposide, adriamycin, vincristine, cyclophosphamide, ifosfamide, cisplatin, vinorelbine, paclitaxel, docetaxel, gemcitabine and pemetrexed.

In the present disclosure, the muscle disease may be selected from the group consisting of sarcopenia and muscular atrophy.

In the present disclosure, the pharmaceutical composition may suppress muscle loss and promote the proliferation of myoblasts and differentiation into muscle cells.

The present disclosure also provides a food composition for preventing or improving a muscle disease, comprising the albumin nanocomposite.

### [Advantageous Effects]

The albumin nanocomposite comprising a phytochemical according to the present disclosure may effectively deliver the phytochemical substance by targeting immune cells that induce reactive oxygen species and inflammatory responses, thereby regulating muscle loss signaling pathways induced by oxidative stress, inflammatory responses, or anticancer agents, suppressing the atrophy of muscle cells and promoting differentiation. Accordingly, the albumin nanocomposite comprising a phytochemical according to the present disclosure may improve muscle loss and prevent and treat muscular atrophy, and thus may be provided as an excellent composition for improving muscle diseases.

### [Description of Drawings]

FIG. 1 is a diagram illustrating an albumin nanocomposite loaded with apigenin according to one embodiment of the present disclosure.
FIG. 2 shows UV analysis results verifying the number of apigenin molecules bound to the surface of glycated albumin according to one embodiment of the present disclosure.
FIG. 3 shows UV analysis results evaluating the release profile of the drug over time and verifying the stability of glycated albumin loaded with apigenin according to one embodiment of the present disclosure.
FIG. 4 shows results confirming the hydrogen peroxide scavenging ability of apigenin-glycated albumin to verify the ROS scavenging effect according to one embodiment of the present disclosure.
FIG. 5 shows results confirming the superoxide radical scavenging ability of apigenin-glycated albumin to verify the ROS scavenging effect according to one embodiment of the present disclosure.
FIG. 6 shows results verifying the cellular fluorescence intensity of glycated albumin (Alb-DBCO-Glc) labeled with FNR648 and non-glycated albumin (Alb-DBCO) to confirm targeting of M1-type macrophages by glucose-glycated albumin according to one embodiment of the present disclosure.
FIG. 7 shows results obtained by treating polarized Raw cell lines (from M0 to M1 state) with FNR648-labeled glycated albumin (Glc-Alb), non-glycated albumin (DBCO-Alb), apigenin alone (Apigenin), apigenin-conjugated non-glycated albumin (AA), and apigenin-conjugated glycated albumin (GA) to confirm selective uptake of glycated albumin by M1-type macrophages, and analyzing (a) cellular morphology and (b) intra-/extracellular ROS levels.
FIG. 8 shows immunofluorescence staining results verifying the cytotoxicity of apigenin-glycated albumin according to one embodiment of the present disclosure, where FIG. 8a shows fluorescence of nuclei stained with DAPI and FIG. 8b presents a graph showing the number of DAPI-stained nuclei.
FIG. 9 shows immunofluorescence staining results verifying the muscle differentiation-promoting effect of apigenin-glycated albumin according to one embodiment of the present disclosure, where FIG. 9a shows immunofluorescence of MHC-positive myotubes visualized in green and DAPI-stained nuclei in blue, and FIG. 9b presents a graph showing the level of myotube differentiation based on total nuclear count.
FIG. 10 shows immunofluorescence staining results verifying the muscle atrophy improvement effect of apigenin-glycated albumin according to one embodiment of the present disclosure, where FIG. 10a shows fluorescence images of myotube differentiation after treating muscle-atrophied cells (induced by cancer cell-conditioned medium) with apigenin alone or apigenin-glycated albumin, and FIG. 10b presents a graph showing the level of myotube differentiation.
FIG. 11 shows Western blot analysis results verifying the effect of apigenin-glycated albumin on the expression of genes related to muscle synthesis and degradation according to one embodiment of the present disclosure.
FIG. 12 shows results verifying the effect of apigenin-glycated albumin on improving muscle atrophy induced by administration of an anticancer agent according to one embodiment of the present disclosure, where FIG. 12a shows fluorescence images of myotube differentiation in mouse muscle cells treated with cisplatin and then with apigenin alone or apigenin-glycated albumin, and FIG. 12b presents a graph showing the level of myotube differentiation.
FIG. 13 shows Western blot analysis results verifying the effect of apigenin-glycated albumin on the expression of myogenin, a factor involved in muscle generation, using proteins extracted from each experimental group in FIG. 12 according to one embodiment of the present disclosure.
FIG. 14 shows fluorescence analysis results verifying the potential of the GLUT-targeting albumin nanocomposite as an injectable formulation, by measuring the remaining amount of albumin nanoplatform over time at the injection site after direct administration into an animal model according to one embodiment of the present disclosure.
FIG. 15 sequentially shows SPECT and PET images of mice intramuscularly injected with apigenin (^{l25}I-Api), apigenin-albumin composite (¹²⁵I-Api/⁶⁴Cu-Alb), and apigenin-glycated albumin composite (¹²⁵I-Api/⁶⁴Cu-Glc-Alb).
FIG. 16a shows fluorescence analysis results verifying myotube differentiation after treating muscle-atrophied cells (induced by cancer cell-conditioned medium) with ursolic acid alone or ursolic acid-glycated albumin, and FIG. 16b presents a graph showing the level of myotube differentiation.
FIG. 17a shows fluorescence analysis results verifying myotube differentiation after treating muscle-atrophied cells with soyasapogenol B alone or soyasapogenol B-glycated albumin, and FIG. 17b presents a graph showing the level of myotube differentiation.
FIG. 18a shows fluorescence analysis results verifying myotube differentiation after treating muscle-atrophied cells with citrusinol alone or citrusinol-glycated albumin, and FIG. 18b presents a graph showing the level of myotube differentiation.

### [Best Mode for Invention]

Hereinafter, specific embodiments of the present disclosure will be described in more detail. Unless otherwise defined, all technical and scientific terms used herein have the same meanings as commonly understood by those skilled in the art to which the present disclosure pertains. In general, the nomenclature used herein is well-known and commonly used in the art.

The present disclosure relates to an albumin nanocomposite comprising a phytochemical and a composition for improving muscle disease comprising the same.

The term "phytochemical" is a compound name combining "phyto," meaning plant-based, and "chemical," and refers to plant-derived chemical substances having physiological activity beneficial to health.

In the present disclosure, by loading a phytochemical onto an albumin nanoplatform, oxidative stress and inflammatory responses induced by anticancer agents or immune cells can be specifically regulated, thereby exhibiting effects of improving muscle diseases related to oxidative stress and inflammatory responses.

In the present disclosure, the phytochemical may be a phenolic compound or a triterpene.

The phenolic compounds usable in the present disclosure preferably include flavonoids, stilbenoids, caffeic acid, p-coumaric acid, curcumin, and resveratrol.

The flavonoids are a type of secondary metabolite produced by plants or fungi and generally have a C15 carbon skeleton composed of two phenyl rings and one heterocyclic ring. More than 5,000 types of natural flavonoids have been identified, and various studies utilizing their diverse activities are ongoing.

The flavonoids may include, but are not limited to, apigenin, citrusinol, flavone, flavonol, flavanone, flavanol, isoflavone, anthocyanins, chrysin, tectochrysin, primetin, acacetin, luteolin, tangeritin, quercetin, kaempferol, genistein, daidzein, naringenin, hesperetin, cyanidin, catechin, and epigallocatechin gallate, with apigenin and citrusinol being preferred.

Apigenin is a type of flavonoid that is a yellow pigment found in various fruits and vegetables and is known to be abundant in parsley, chamomile, celery, and carrots. Traditionally, apigenin has been consumed for centuries in the form of chamomile tea or applied topically to the skin. More recently, it has been identified as a molecule capable of regulating various intracellular mechanisms and is particularly known to inhibit inflammation and oxidative stress and enhance carbohydrate metabolism.

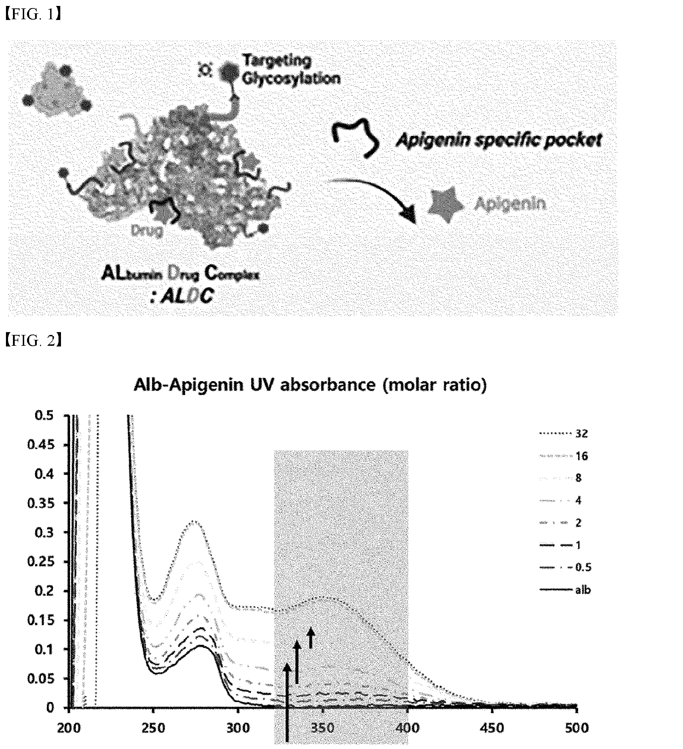

In the present disclosure, as shown in FIG. 1, apigenin is loaded onto an albumin-based nanocomposite, and such configuration enables targeting of activated M1-type inflammatory macrophages around acute inflammation or tumor sites and accurate delivery of apigenin to the targeted location. Through this, an albumin nanocomposite and a composition comprising the same can be provided to suppress muscle loss induced by oxidative stress or inflammatory responses and to promote the differentiation of muscle cells, thereby improving muscle diseases.

Citrusinol is a flavonoid compound found in plants such as Citrus unshiu and Phyllodium pulchellum. According to the present disclosure, by loading citrusinol onto an albumin nanoplatform, an albumin nanocomposite capable of improving muscle disease can be provided.

Triterpenes are a class of compounds among terpenes that constitute the main components of plant-based oils and have a carbon skeleton composed of 30 carbon atoms. Most triterpenes have tetracyclic or pentacyclic structures. Triterpenes are known to have anti-inflammatory, antioxidant, and anticancer activities.

In the present disclosure, the triterpene may be at least one selected from the group consisting of ursolic acid, soyasapogenol A, soyasapogenol B, ursane, oleanane, lupane, saponin, sterol, oleanolic acid, lupeol, corosolic acid, maslinic acid, betulinic acid, ginsenoside, beta-sitosterol, campesterol, stigmasterol, lanosterol, and germanicol, with ursolic acid and soyasapogenol B being preferred.

The phytochemical substance delivered by the albumin nanocomposite of the present disclosure may exert an effect of improving muscle diseases related to oxidative stress and inflammatory responses by specifically regulating oxidative stress and inflammatory responses induced by anticancer agents or immune cells. In particular, the albumin nanocomposite of the present disclosure not only improves the low water solubility and delivery efficiency of natural active ingredients such as apigenin, but also exhibits superior effects compared to treatment with the phytochemical substance alone by effectively scavenging intracellular reactive oxygen species (ROS). These effects have been experimentally demonstrated at the immune cell level. Furthermore, upon treatment in muscle cells, it was confirmed that the expression levels of proteins associated with muscle cell differentiation were increased and that muscle cell differentiation was promoted, thereby verifying the anti-inflammatory effect and the resulting improvement in muscle function.

One aspect of the present disclosure provides an albumin nanocomposite, which is obtained by a click chemistry reaction between albumin conjugated with an azide (N₃) or cyclooctyne functional group and a transmitter conjugated with an azide (N₃) or cyclooctyne functional group, wherein a phytochemical is loaded on the surface of the albumin, the transmitter comprises a glucosyl group, and when the albumin is conjugated with the azide functional group, the transmitter is conjugated with the cyclooctyne functional group, and when the albumin is conjugated with the cyclooctyne functional group, the transmitter is conjugated with the azide functional group.

In the present disclosure, the albumin refers to a protein that constitutes the basic material of cells, is abundantly present in blood, and is produced in the liver. The albumin has the smallest molecular weight among naturally occurring simple proteins. Serum albumin in the blood serves to maintain and restore plasma volume, thereby preventing shock caused by excessive bleeding, and is used in surgeries and burn treatments. It is also known to have oxygen transport capacity similar to that of hemoglobin.

The albumin may include any albumin capable of being formulated, but is preferably derived from human plasma or recombinant human serum albumin produced by genetic engineering, though not limited thereto. Genetic information regarding the albumin of the present disclosure may be obtained from publicly available databases such as NCBI GenBank.

In the present disclosure, the number of amino groups (-NH₂) exposed on the surface of the albumin may range from 15 to 30.

In a specific embodiment of the present disclosure, in order to prepare a nanocomposite capable of targeting in vivo target tissues based on biocompatible albumin and delivering a transmitter such as a drug or fluorescent material to exert a therapeutic effect on diseases, human serum albumin (HSA), a representative biocompatible albumin, was used. Cyclooctyne or azide (N₃), which is one of the functional groups used in click chemistry, was introduced onto the surface of HSA under reaction conditions that minimize denaturation of HSA.

In the present disclosure, azide (N₃) is a reactive group composed of three nitrogen atoms and is known to have high reactivity. In particular, it serves as an electron donor in a 1,3-dipolar cycloaddition reaction, which is a type of Cu-free click chemistry, and plays a role in forming a triaza-5-membered ring.

The cyclooctyne functional group is an eight-membered aliphatic ring containing a triple bond under ring strain and is known to act as an electron acceptor in a 1,3-dipolar cycloaddition reaction, which is a type of Cu-free click chemistry, thereby forming a triaza-5-membered ring. Due to the structural characteristics of cyclooctyne, namely, the ring-strained triple bond structure, click chemistry can be performed without a Cu(I) catalyst.

The cyclooctyne functional group may be at least one selected from the group consisting of 4-cyclooctyn-1-yl 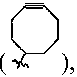 3-cyclooctyn-1-yl 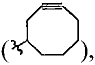 2-cyclooctyn-1-yl 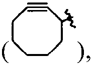 monofluorinated cyclooctyne (MOFO) 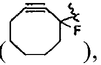 difluorinated cyclooctyne (DIFO) 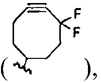 dimethoxyazacyclooctyne (DIMAC) 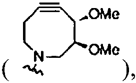 dibenzocyclooctyne (DIBO) 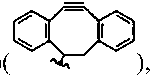 azadibenzocyclooctyne (ADIBO) 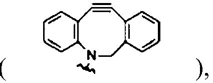 and biarylazacyclooctynone (BARAC) 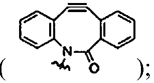 however, it is not necessarily limited thereto.

In the present disclosure, the number of click reaction functional groups (azide or cyclooctyne functional groups) introduced onto the surface of the albumin is preferably from 1 to 14, and more preferably from 9 to 12. In this case, a variety of transmitters can be introduced into the body in excess, thereby increasing the likelihood of uptake at the target site. In contrast, if the number of click reaction functional groups exceeds the above range, the nanocomposite may be immediately taken up by the liver upon administration into the body, limiting uptake at other target disease sites. The number of click reaction functional groups introduced onto the albumin surface may be controlled according to the reaction ratio of albumin to azide-NHS or cyclooctyne-NHS.

A solution in which azide (N₃) is conjugated or a solution in which albumin is conjugated with a cyclooctyne functional group may be obtained by (a) dissolving albumin in a phosphate-buffered saline (PBS), (b) dissolving azide-NHS or cyclooctyne-NHS in DMSO, and (c) mixing the obtained solutions and then reacting the mixture at 20°C to 37°C for 30 minutes to 1 hour.

In step (a), the phosphate-buffered saline (PBS) may have a pH ranging from 6.8 to 7.6, and preferably from 7.0 to 7.4.

In step (b), the amount of DMSO used to prepare the azide-NHS solution or cyclooctyne-NHS solution may be 2% (v/v) or less of the total reaction solution.

In step (c), the molar mixing ratio of albumin to azide-NHS or cyclooctyne-NHS may range from 1:1 to 1:25.

In step (c), when mixing the albumin solution with the azide-NHS solution, the functional group conjugated to the albumin may be an azide functional group, and when mixing the albumin solution with the cyclooctyne-NHS solution, the functional group conjugated to the albumin may be a cyclooctyne functional group.

In one embodiment of the present disclosure, a human serum albumin (HSA) solution was mixed with an ADIBO-NHS solution and reacted at 37°C for 30 minutes to prepare HSA-ADIBO.

The number of click reaction functional groups (azide or cyclooctyne functional groups) introduced onto the surface of the albumin is preferably from 1 to 14, and more preferably from 10 to 12. In this case, various transmitters can be introduced into the human body in excess, thereby increasing the likelihood of uptake at the target site. In contrast, if the number of click reaction functional groups exceeds the above range, the nanocomposite may be immediately taken up by the liver upon administration into the body, limiting uptake at other target disease sites. The number of click reaction functional groups introduced onto the albumin surface may be controlled according to the reaction ratio of albumin to azide-NHS or cyclooctyne-NHS.

The albumin nanocomposite of the present disclosure is obtained by mixing a solution containing albumin conjugated with an azide (N₃) or cyclooctyne functional group and a solution containing a transmitter conjugated with an azide (N₃) or cyclooctyne functional group, and performing a click chemistry reaction, wherein the click chemistry reaction may be a copper catalyst-free (Cu-free) click chemistry reaction.

In one aspect of the present disclosure, since the azide functional group used as a click chemistry functional group acts as an electron donor and the cyclooctyne functional group acts as an electron acceptor, it is preferable that when the functional group conjugated to albumin is an azide functional group, the functional group conjugated to the transmitter is a cyclooctyne functional group, and when the functional group conjugated to albumin is a cyclooctyne functional group, the functional group conjugated to the transmitter is an azide functional group.

In the present disclosure, the albumin nanocomposite may comprise a phytochemical on the surface of the albumin.

In this case, the phytochemical loaded into the nanocomposite refers to the previously listed phytochemical substances and their derivatives. For example, a derivative of apigenin may refer to a compound in which a portion of the oxygen or hydrogen atoms included in apigenin is substituted with other atoms or substituents.

The phytochemical is loaded on the surface of the albumin, and it is analyzed that there exists a specific pocket on the albumin surface capable of selectively accommodating the phytochemical, thereby allowing the phytochemical to be stably loaded and forming the nanocomposite.

The nanocomposite of the present disclosure may deliver the phytochemical loaded on the albumin surface accurately to the target site, thereby suppressing muscle loss induced by oxidative stress, inflammatory response, or administration of anticancer agents, and promoting the differentiation of muscle cells, thus improving muscle diseases.

In particular, when the albumin nanocomposite loaded with apigenin is used in the form of glycated albumin as described later, it may exhibit a synergistic effect in muscle disease improvement activity.

The nanocomposite may comprise 1 to 20 phytochemicals on the surface of the albumin, more preferably 2 to 10 phytochemicals, and still more preferably 4 to 8 phytochemicals.

The albumin nanocomposite of the present disclosure, having multiple click reaction functional groups, allows various transmitters to be conjugated to the multiple reactive sites.

In the present disclosure, the transmitter refers to a substance that is conjugated with albumin and delivered into the body, and the transmitter comprises a glucosyl group, the structure of which is shown in Formula 1 below.

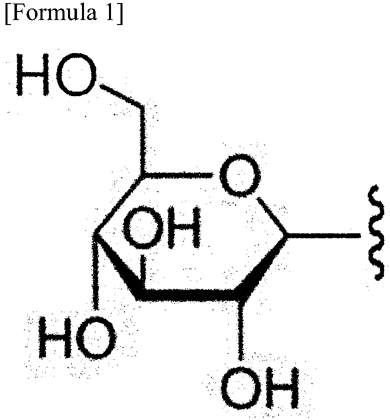

In the present disclosure, the albumin nanocomposite delivers the phytochemical by targeting GLUT (Glucose Transporter), which is overexpressed on M1-type macrophages, using glucose as a targeting ligand.

In the present disclosure, the albumin nanocomposite may comprise 4 to 8 glucosyl groups, and more preferably 5 to 7 glucosyl groups. In this case, the nanocomposite administered into the body may remain for an extended period at the injection site or in the bloodstream, thereby increasing the targeting potential to the target site. However, when the number of glucosyl groups exceeds 8, the nanocomposite may be immediately taken up by the liver upon administration, thus limiting targeting to the disease site.

In the present disclosure, the transmitter, i.e., the glucosyl group, may be conjugated to the albumin through a click chemistry reaction between the azide or cyclooctyne functional group conjugated to albumin and the azide or cyclooctyne functional group conjugated to the glucosyl group.

At this time, GLUT, which is overexpressed on the surface of M1-type cells, recognizes OH functional groups at the 1st, 3rd, and 5th positions of glucose in order to acquire glucose as an energy source. In the present disclosure, in order to more effectively target M1-type macrophages, albumin may be conjugated at the 1st, 2nd, and 6th carbon positions of glucose, with the 6th position being particularly preferred.

For example, glucosyl groups conjugated with azide at the 1st, 2nd, or 6th carbon positions correspond to the chemical structures shown in Formula 2, Formula 3, and Formula 4, respectively.

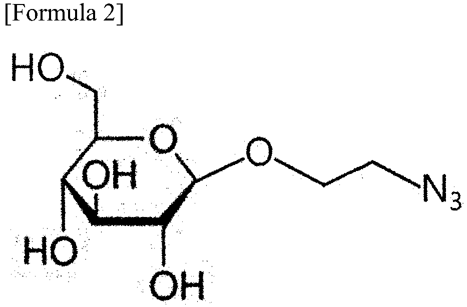

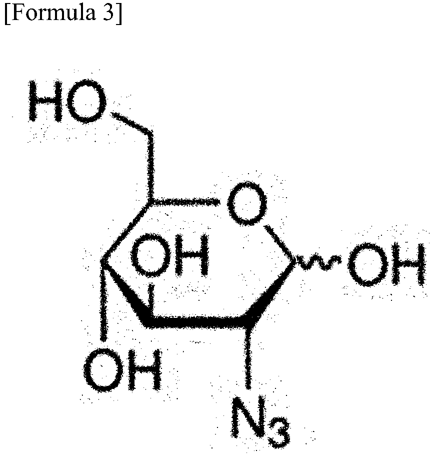

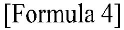

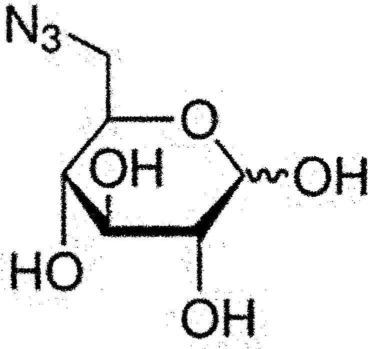

A nanocomposite in which albumin is conjugated at the 6th carbon position of glucose may exhibit GLUT targeting ability that is three times and two times higher, respectively, than nanocomposites in which albumin is conjugated at the 1st and 2nd carbon positions of glucose.

In the present disclosure, the nanocomposite may further comprise at least one additional substance as a transmitter, and in this case, multiple substances may be conjugated to the albumin conjugated with the azide or cyclooctyne functional group simultaneously via click chemistry, or each substance may be conjugated sequentially via click chemistry.

In the present disclosure, the transmitter may further comprise a radioactive isotope. The radioactive isotope refers to an element having the same atomic number but a different atomic mass, and among such isotopes, those that are radioactive are referred to as radioactive isotopes. These radioactive isotopes may be used as important markers for disease diagnosis or pharmacokinetic analysis by utilizing their property of attenuating radioactivity through the emission of gamma rays or other subatomic particles. In the present disclosure, the radioactive isotope usable as a marker is not limited as long as it is known in the art, and may be at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹Fe, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn, and ¹⁷⁷Lu, and preferably ¹¹C, ¹⁸F, ⁶⁴Cu, ⁶⁷Ga, ⁶⁸Ga, ⁸⁹Zr, ^{99m}Tc, ¹¹¹In, and ¹²³I, but is not necessarily limited thereto.

In the present disclosure, the radioactive isotope may be labeled by a chelating agent, which serves to connect the radioactive isotope to the albumin. The chelating agent may be at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N2S2 (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino]-6,11,17,22-tetraazaheptaeicosane]thiourea), and HYNIC (hydrazinonicotinic acid), but is not necessarily limited thereto.

In the present disclosure, the transmitter may further comprise a fluorescent material.

The fluorescent material refers to a substance that emits specific wavelengths of visible light in response to specific wavelengths of excitation light, and in the present disclosure, includes any fluorescent material that may be conjugated to the albumin nanocomposite and used to identify the location of the nanocomposite.

Specifically, the fluorescent material usable as a marker in the present disclosure may be used without limitation so long as it is known in the art, and may include, but is not limited to: rhodamine-based materials including rhodamine and TAMRA (tetramethylrhodamine); fluorescein-based materials including fluorescein, FITC (fluorescein isothiocyanate), and FAM (fluorescein amidite); bodipy (boron-dipyrromethene); Alexa Fluor dyes; and cyanine-based fluorescent materials including Cy3, Cy5, Cy7, and indocyanine green.

Another aspect of the present disclosure provides a pharmaceutical composition for preventing or treating a muscle disease, comprising the albumin nanocomposite comprising a phytochemical.

In the present disclosure, the muscle disease may be a muscle disease induced by an anticancer agent or an inflammatory response.

Specifically, the albumin nanocomposite comprising a phytochemical according to the present disclosure may suppress muscle loss by regulating signaling pathways that induce muscle weakening due to oxidative stress or inflammatory response induced by administration of anticancer agents or immune cells, and may promote the differentiation of muscle cells to induce an increase in muscle mass.

According to one embodiment of the present disclosure, the glycated albumin nanocomposite loaded with apigenin selectively targeted inflammatory M1-type macrophages in mouse muscle cells, suppressed the reduction of muscle cells caused by inflammatory response, and promoted the differentiation of myoblasts. In addition, it suppressed the reduction and atrophy of muscle cells induced by administration of anticancer agents and promoted the differentiation of muscle cells, thereby exhibiting an effect of improving muscle loss and muscular atrophy.

In particular, compared to the group treated with apigenin alone, the group treated with the glycated albumin nanocomposite loaded with apigenin exhibited a significant reduction in ROS and inhibition of differentiation into the inflammatory type of macrophages. Furthermore, even in muscle cells with atrophy induced by cancer cell-conditioned medium and anticancer agents, the improvement effect in the group treated with the glycated albumin nanocomposite loaded with apigenin was significantly higher than that in the group treated with apigenin alone, thereby confirming that the combination of apigenin and glycated albumin nanocomposite of the present disclosure exhibits a synergistic effect.

Additionally, it was confirmed that the albumin nanocomposite comprising apigenin suppresses the expression of genes involved in muscle cell degradation induced by immune cells and administration of anticancer agents, and increases the expression of genes that induce differentiation of myoblasts into muscle cells. Therefore, the pharmaceutical composition comprising the albumin nanocomposite comprising apigenin according to the present disclosure may suppress muscle loss and promote proliferation of myoblasts and differentiation into muscle cells, thereby preventing or treating muscle diseases.

In the present disclosure, the anticancer agent may be at least one selected from the group consisting of etoposide, adriamycin, vincristine, cyclophosphamide, ifosfamide, cisplatin, vinorelbine, paclitaxel, docetaxel, gemcitabine, and pemetrexed.

In another embodiment of the present disclosure, it was confirmed that ursolic acid, soyasapogenol B, and citrusinol, when formulated as albumin nanocomposites in the same manner as apigenin, were capable of suppressing muscle loss and promoting proliferation of myoblasts and differentiation into muscle cells, and in particular, exhibited superior synergistic effects compared to when these substances were used alone.

In the present disclosure, the muscle disease may be selected from the group consisting of sarcopenia and muscular atrophy.

In the present disclosure, the sarcopenia or muscular atrophy includes not only the dictionary meanings of sarcopenia and muscular atrophy, but also muscle diseases that cause or are derived from muscle loss or muscle atrophy, such as sarcopenic obesity, myopathy, muscular dystrophy, muscular injury, myasthenia, myoneural conductive disease, nerve injury, amyotrophic lateral sclerosis (ALS), atony, myotonia, and cachexia.

For example, the muscular atrophy may include diabetic amyotrophy and spinal muscular atrophy, and the muscular dystrophy may include Duchenne muscular dystrophy, Becker muscular dystrophy, limb girdle muscular dystrophy, facioscapulohumeral muscular dystrophy, oculopharyngeal muscular dystrophy, and myotonic dystrophy. The myopathy may include inflammatory myopathies such as polymyositis and dermatomyositis, endocrine myopathy, toxic myopathy, metabolic myopathy, mitochondrial myopathy, and congenital myopathy.

The pharmaceutical composition of the present disclosure may further comprise an appropriate pharmaceutically acceptable carrier, excipient, or diluent according to conventional methods. The pharmaceutically acceptable carrier is commonly used in formulation preparation and may include, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

The pharmaceutical composition of the present disclosure may further comprise lubricants, wetting agents, sweeteners, flavoring agents, emulsifiers, suspending agents, preservatives, and the like. Suitable pharmaceutically acceptable carriers and formulation methods may be preferably selected and formulated for each component using the methods disclosed in Remington's Pharmaceutical Sciences (19th ed., 1995).

The pharmaceutical composition of the present disclosure may be administered orally or parenterally. Parenteral administration includes intravenous injection, subcutaneous injection, intramuscular injection, intraperitoneal injection, and transdermal administration.

Oral formulations may include, for example, tablets, pills, hard or soft capsules, liquid solutions, suspensions, emulsions, syrups, and granules. These formulations may additionally comprise, in addition to the active ingredient, diluents such as lactose, dextrose, sucrose, mannitol, sorbitol, cellulose and/or glycine, and lubricants such as silica, talc, stearic acid and its magnesium or calcium salts and/or polyethylene glycol. The tablets may also comprise binders such as magnesium aluminum silicate, starch paste, gelatin, tragacanth, methyl cellulose, sodium carboxymethyl cellulose, and/or polyvinylpyrrolidone. In certain cases, disintegrants such as starch, agar, alginic acid or its sodium salts, effervescent mixtures, and/or adsorbents, coloring agents, flavoring agents, and sweeteners may also be included. These formulations may be prepared by conventional mixing, granulation, or coating methods.

Additionally, a representative parenteral formulation is an injectable formulation, and the solvent for the injectable formulation may include water, Ringer's solution, isotonic saline, or suspensions.

Sterile fixed oils may also be used as solvents or suspension media for the injectable formulation, and any non-irritant fixed oil including mono- and diglycerides may be used for this purpose. Fatty acids such as oleic acid may also be used.

The composition according to the present disclosure is administered in a pharmaceutically effective amount. In the present disclosure, the term "pharmaceutically effective amount" refers to an amount sufficient to treat a disease with a reasonable benefit/risk ratio applicable to medical treatment. The effective dosage level may be determined according to factors such as the type and severity of the disease, activity of the drug, sensitivity to the drug, timing of administration, route of administration, rate of excretion, treatment duration, concomitant medications, and other factors known in the medical field. The composition of the present disclosure may be administered as a single therapy or in combination with other therapies, either sequentially or simultaneously with conventional therapies, and may be administered in a single or multiple dose regimen. It is important to administer the minimum amount necessary to achieve maximum efficacy without side effects by considering all the above factors, which can be readily determined by a person of ordinary skill in the art.

Specifically, the effective amount of the composition of the present disclosure may vary depending on the age, sex, and body weight of the patient, and in general, 0.001 to 150 mg per kg of body weight, preferably 0.01 to 100 mg, may be administered daily or every other day, or may be administered one to three times per day in divided doses. However, the dosage may be increased or decreased depending on the route of administration, sex, body weight, age, and other factors, and the dosage described above does not limit the scope of the present disclosure in any way.

The present disclosure also provides a food composition for preventing or improving a muscle disease, comprising the nanocomposite comprising a phytochemical. The food of the present disclosure may include not only food for humans but also animal feed and feed additives.

In another specific embodiment of the present disclosure, the food composition may further comprise at least one additive selected from the group consisting of organic acids, phosphates, antioxidants, lactose casein, dextrin, glucose, sugar, and sorbitol. The organic acid may include, but is not limited to, citric acid, fumaric acid, adipic acid, lactic acid, or malic acid. The phosphate may include, but is not limited to, sodium phosphate, potassium phosphate, acid pyrophosphate, or polyphosphate (condensed phosphate). The antioxidant may include, but is not limited to, natural antioxidants such as polyphenols, catechins, α-tocopherol, rosemary extract, licorice extract, chitosan, tannic acid, or phytic acid.

In another specific embodiment of the present disclosure, the food composition may further comprise, in addition to the active ingredient, various nutrients, vitamins, minerals (electrolytes), synthetic flavoring agents, natural flavoring agents, coloring agents, bodying agents (e.g., cheese, chocolate), pectic acid and salts thereof, alginic acid and salts thereof, organic acids, protective colloid thickeners, pH regulators, stabilizers, preservatives, glycerin, alcohols, and carbonation agents used in carbonated beverages. The food composition according to one embodiment of the present disclosure may also contain fruit pulp for the production of natural fruit juice, fruit juice beverages, or vegetable beverages.

According to one embodiment of the present disclosure, the formulation of the food composition may be, but is not limited to, in the form of solid, powder, granule, tablet, capsule, liquid, or beverage.

The food composition may be used in the manufacture of food products including, but not limited to, confectionery, candies, ice cream products, dairy products, meat products, fish products, tofu or jelly products, edible oils and fats, noodles, teas, beverages, special nutritional foods, health supplements, seasoned foods, ice, ginseng products, pickled kimchi products, dried fruits, fruits, vegetables, dried or cut fruit and vegetable products, fruit juice, vegetable juice, mixed juices thereof, chips, noodle products, processed livestock foods, processed seafood products, dairy-based foods, fermented milk products, legume-based foods, grain-based foods, microbially fermented foods, bakery and confectionery, seasonings, processed meat products, acidic beverages, licorice products, and herbal products.

As described above, the albumin nanocomposite comprising a phytochemical according to the present disclosure may effectively deliver the phytochemical by targeting immune cells that induce reactive oxygen species and inflammatory responses, thereby regulating signaling pathways related to muscle loss induced by oxidative stress and inflammatory responses, suppressing the atrophy of muscle cells, and promoting differentiation, thus improving muscle loss and exerting preventive and therapeutic effects against muscular atrophy.

### Examples

The present disclosure will be described in more detail by way of the following examples. However, these examples are provided solely for illustrative purposes to explain certain experimental methods and compositions of the present disclosure, and the scope of the present disclosure is not limited to these examples.

### Preparation Example 1: Preparation of Apigenin-Glycated Albumin Composite

### 1-1. Preparation of Glycated Albumin Composite

Glycated albumin with conjugated glucosyl groups was prepared as follows.

Albumin was dissolved in phosphate-buffered saline (PBS, pH 7.4) at a concentration of 40 mg/mL, and 20 mg/0.5 mL of the albumin solution was aliquoted into each vial. DBCO-NHS was dissolved in DMSO (10 mg/50 µL) and added to the albumin solution to make a total of 500 µL.

Albumin and DBCO-NHS were reacted at a molar ratio of 1:14 at room temperature for 6 hours, and the resulting mixture was purified using an Amicon Ultra-0.5 centrifugal filter tube to obtain DBCO-albumin (DBCO-Alb).

For the synthesis of glucose-albumin, 6-azido-glucose was dissolved in distilled water at a concentration of 3 mg/mL, then added to the DBCO-Alb solution (4 mg/0.5 mL), incubated at room temperature for 1 hour, and purified using a centrifugal filter tube.

Next, UV-Vis spectroscopy was used to confirm the absorbance trends at 280 and 309 nm for glucose-DBCO-Alb (Alb-DBCO-Glc). Each albumin sample was diluted to 2 mg/mL for absorbance measurements. The number of ADIBO and glucose molecules bound to the albumin was determined using MALDI-TOF MS (degree of functionalization = DOF).

Subsequently, the number of DBCO molecules on the albumin surface and the number of glucose molecules introduced via click reaction were quantified using UV and MALDI-TOF analysis. To prepare DBCO¹¹-Alb-FL and Glc⁶-DBCO¹¹-Alb-FL, N₃-FNR648 (10 nmol/1 µL, dissolved in DMSO) was added to DBCO-Alb and Glc-DBCO-Alb (30 nmol/500 µL), incubated at 4°C for 30 minutes, and purified using a centrifugal filter unit.

As a result of quantification of the number of DBCO functional groups introduced into HSA-DBCO using UV-Vis spectroscopy, 12.8 to 13.1 groups were confirmed, and by MALDI-TOF analysis, 11.1 to 11.5 groups were confirmed. The number of conjugated glucose molecules was found to be 5.4 to 7.8.

### 1-2. Preparation of Apigenin-Loaded Glycated Albumin Composite

Apigenin was loaded onto the glycated albumin prepared as in Preparation Example 1-1.

Apigenin was mixed with albumin at 0.5, 1, 2, 4, 8, 16, and 32 molar equivalents based on albumin, and reacted for 0.5 to 2 hours, followed by purification using a filter and evaluation via UV spectroscopy. As shown in FIG. 2, a characteristic apigenin peak in the range of 350 to 380 nm saturated at around 16 equivalents, indicating that approximately 8 to 16 apigenin molecules were bound per albumin.

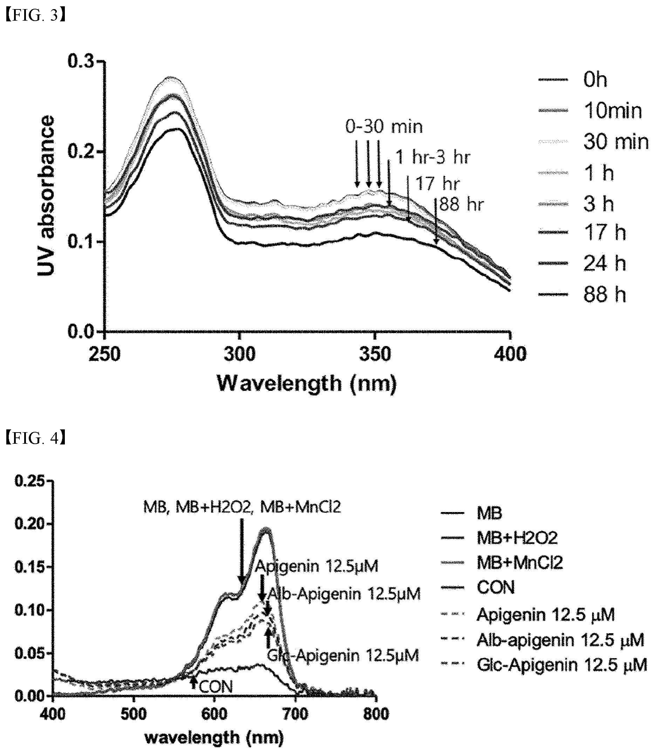

Next, to assess the stability of the composite, the drug release over time was evaluated. The moment of preparation of the apigenin-loaded glycated albumin was designated as 0 hours, and albumin was sampled at 10 min, 30 min, 1 hr, 1 hr 30 min, 3 hr, 6 hr 50 min, 17 hr, 24 hr, and 88 hr. The release rate was then confirmed by UV spectroscopy and shown in FIG. 3.

As shown in FIG. 3, approximately 20% of apigenin was released after 88 hours, confirming that apigenin was stably loaded onto the glycated albumin to form the apigenin-glycated albumin composite.

### Experimental Example 1: Confirmation of Reactive Oxygen Species (ROS) Scavenging Effect

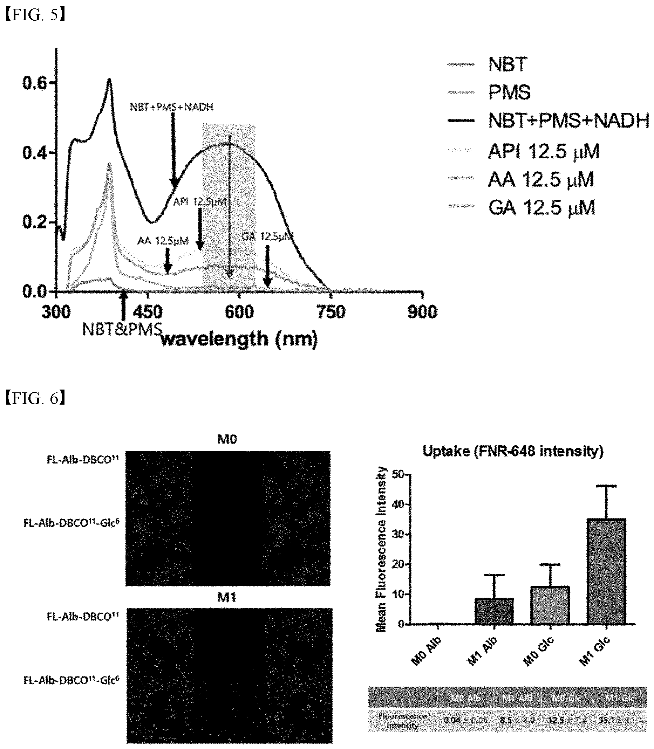

To confirm the ROS scavenging effect of the apigenin-glycated albumin composite prepared in Preparation Example 1, experiments were conducted to evaluate the scavenging ability against hydrogen peroxide and oxygen radicals, with the results shown in FIGs. 4 and 5, respectively.

In the experiment, apigenin alone (API), apigenin bound to albumin (AA), and apigenin bound to glycated albumin (GA) were evaluated and compared, and the apigenin concentration was uniformly maintained at 12.5 µM.

As shown in FIG. 4, which confirmed the hydrogen peroxide scavenging effect, all groups showed a similar level of activity, indicating that the albumin-based composite formation did not affect the ROS scavenging capability of apigenin.

As shown in Figure 5, a similarly effective oxygen radical scavenging effect was also observed.

These results indicate that the apigenin-glycated albumin composite possesses ROS scavenging activity and that the pharmacological efficacy of apigenin is retained even when forming a composite with albumin.

### Experimental Example 2: Confirmation of Targeting and Inhibitory Effect on M1-Type Macrophages

### 2-1. Confirmation of Targeting Ability of Glycated Albumin to M1-Type Macrophages

The glycated albumin prepared in Preparation Example 1-1 was synthesized using glucose as a target ligand to selectively target GLUT (glucose transporter), which is overexpressed in M1-type macrophages.

To verify whether the albumin glycated with glucose can selectively target M1-type macrophages, RAW cells were polarized into M0 and M1 states, followed by treatment with FNR648-labeled glycated albumin (Alb-DBCO-Glc) and non-glycated albumin (Alb-DBCO).

As shown in FIG. 6, glycated albumin exhibited more than fourfold higher uptake in M1 macrophages compared to non-glycated albumin.

Specifically, both types of albumin showed no significant difference in uptake in M0 cells and no significant differences across all groups, however, the fluorescence expression of glycated albumin was significantly increased in M1 cells. This confirmed that glycated albumin serves as a platform selectively taken up by M1-type macrophages.

### 2-2. Confirmation of M1-Type Macrophage Inhibition by Apigenin-Glycated Albumin

It was further examined whether apigenin-glycated albumin can selectively inhibit M1-type macrophages as observed in the previous experiment.

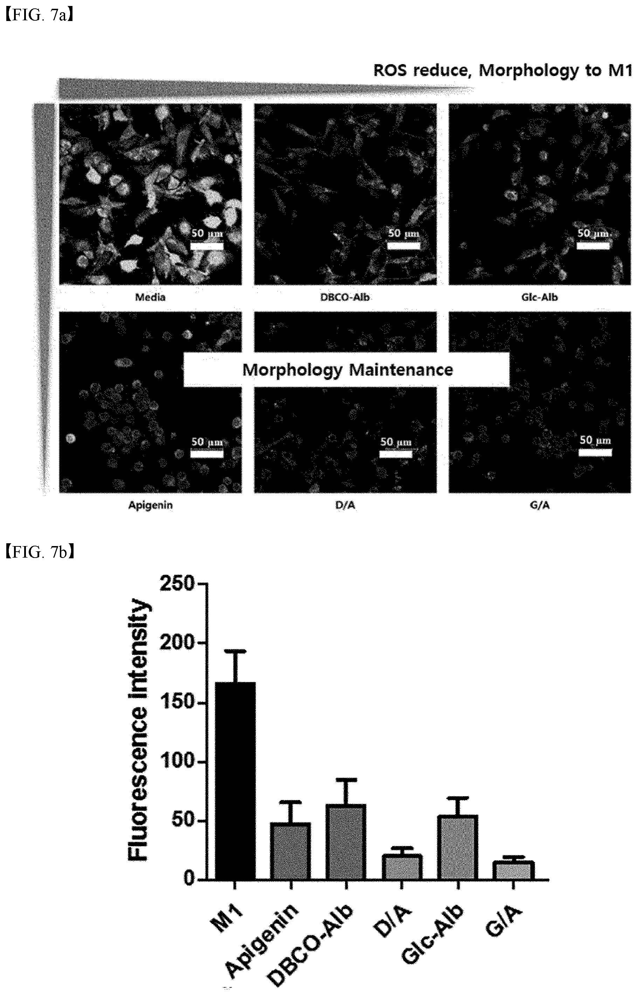

Using the same method as in Experimental Example 2-1, RAW cells were polarized from M0 to M1, and then treated with FNR648-labeled glycated albumin (Glc-Alb), non-glycated albumin (DBCO-Alb), apigenin (Apigenin) alone, apigenin-bound non-glycated albumin (D/A), and apigenin-bound glycated albumin (G/A). The fluorescence expression was then evaluated, along with cell morphology and intracellular/extracellular ROS levels, as shown in FIGS. 7a and 7b, respectively.

As a result, ROS reduction was observed in the groups treated with apigenin alone and apigenin-bound albumin (D/A and G/A), and the RAW cell morphology was maintained in the M0 type.

In particular, the G/A group (apigenin-bound glycated albumin) exhibited a significantly greater reduction in ROS and effectively inhibited polarization to the M1 type compared to other groups, indicating a synergistic effect between apigenin and glycated albumin.

### Experimental Example 3: Cytotoxicity Evaluation of Apigenin-Glycated Albumin

The cytotoxicity of the apigenin-glycated albumin was evaluated using immunofluorescence staining.

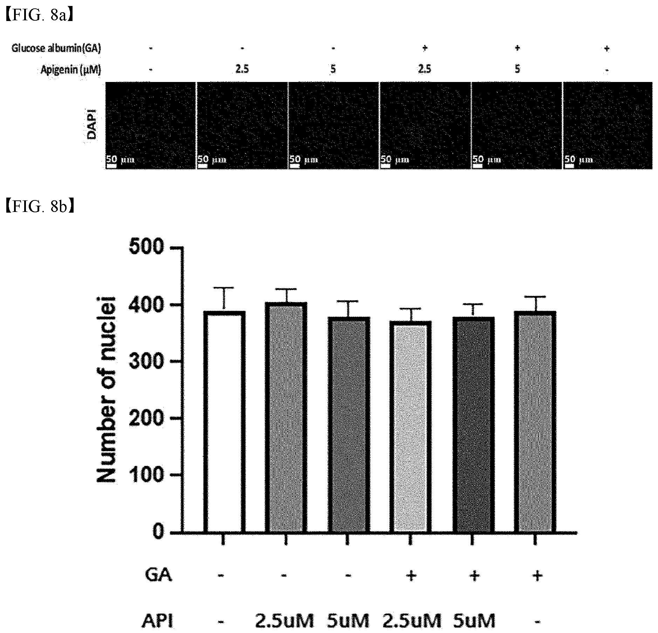

Mouse muscle cells (C2C12) were cultured and treated with either apigenin alone or apigenin-glycated albumin, with apigenin administered at concentrations of 2.5 and 5 µM, respectively. After 96 hours of incubation, cell nuclei were stained with DAPI. Fluorescence expression was observed as shown in FIG. 8a, and the number of stained nuclei was counted and plotted in FIG. 8b.

Referring to FIG. 8b, which shows the number of nuclei from viable cells, neither the apigenin alone nor the apigenin-glycated albumin composite exhibited cytotoxicity.

### Experimental Example 4: Evaluation of Myogenic Differentiation-Promoting Effect of Apigenin-Glycated Albumin

To evaluate the muscle differentiation-promoting effect of the apigenin-glycated albumin, immunofluorescence staining was performed.

Mouse muscle cells (C2C12) were cultured and treated with either apigenin alone or apigenin-glycated albumin, with the apigenin concentration adjusted to 2.5 or 5 µM in both cases. The cells were then differentiated into myotubes over 48 hours.

Next, the cells were fixed using a 4% formaldehyde solution, followed by overnight incubation at 4°C with the primary antibody MHC (myosin heavy chain, Developmental Studies Hybridoma Bank, Iowa City, Iowa, USA) and DAPI (4',6-Diamidino-2-Phenylindole, Dihydrochloride, Sigma Aldrich, MO, USA). The secondary antibody was then applied at room temperature for 1 hour.

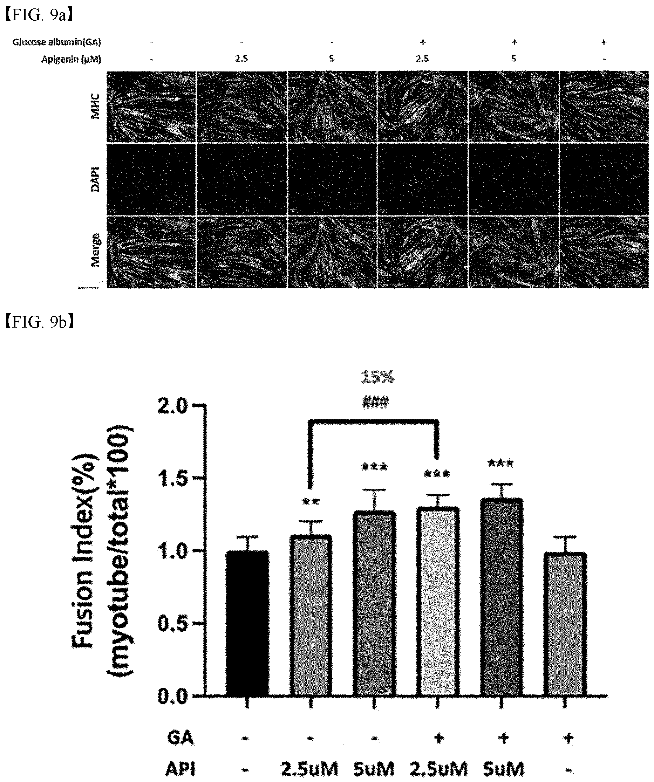

As shown in FIG. 9a, MHC-positive myotubes were visualized in green, and DAPI-stained nuclei were visualized in blue. Myogenic differentiation was quantified using the fusion index (Fusion Index = number of nuclei within myotubes / muscle cells), and the results were displayed in FIG. 9b.

Referring to FIGS. 9a and 9b, the group treated with the apigenin-glycated albumin composite showed greater myotube differentiation compared to the apigenin-only group. In particular, the group treated with 2.5 µM apigenin-glycated albumin exhibited a 15% higher myotube differentiation rate than the group treated with apigenin alone at the same concentration. These findings confirm that the apigenin-glycated albumin enhances myoblast differentiation and promotes muscle formation.

### Experimental Example 5: Evaluation of the Effect of Apigenin-Glycated Albumin on Improvement of Muscle Atrophy

To evaluate the effect of the apigenin-glycated albumin on muscle atrophy improvement, immunofluorescence staining was performed using the same method as in Experimental Example 3.

First, mouse muscle cells (C2C12) were cultured, and the culture medium was replaced with cancer cell-conditioned medium (CCM) from cultured CT26 (colon carcinoma) cells to induce muscle atrophy. The cells were then treated with either apigenin alone or the apigenin-glycated albumin at concentrations of 2.5 µM and 5 µM, and cultured for 48 hours. After incubation, fluorescence expression in the cells was observed to evaluate the level of myotube differentiation.

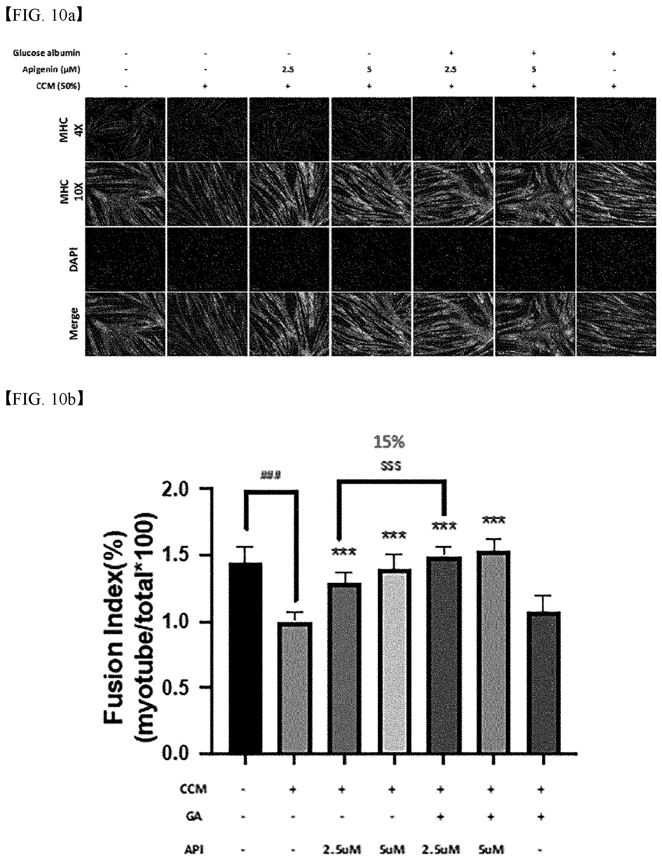

Referring to FIGs. 10a and 10b, which show the level of myotube differentiation in atrophied myoblasts treated with either apigenin alone or the apigenin-glycated albumin, it was confirmed that myotube formation was increased in the group treated with the apigenin-glycated albumin compared to the group treated with apigenin alone. In particular, in the group treated with the 2.5 µM apigenin-glycated albumin, the level of myotube differentiation increased by 15% compared to the group treated with the same concentration of apigenin alone, indicating that the apigenin-glycated albumin composite can enhance myoblast differentiation and promote recovery of atrophied muscle.

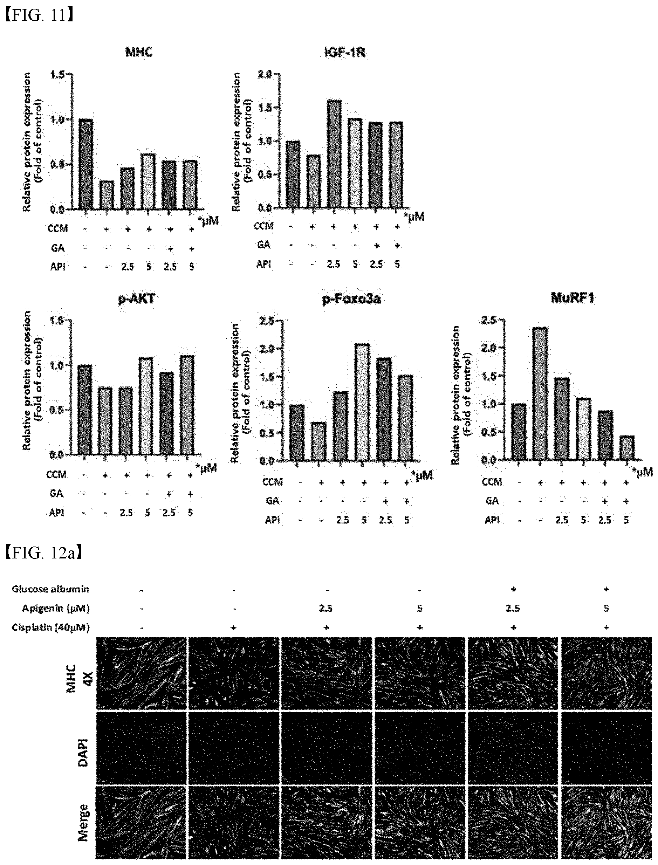

Meanwhile, to evaluate the effect of the apigenin-glycated albumin composite on the expression of genes related to muscle generation and degradation, proteins were extracted from the cells of each experimental group and analyzed via western blotting. The expression levels of each protein were presented in FIG. 11.

As shown in FIG. 11, increased expression of MHC was observed in both the apigenin-treated and apigenin-glycated albumin-treated groups, indicating recovery of muscle cells that had undergone atrophy due to CCM treatment. Additionally, both the apigenin-treated and apigenin-glycated albumin-treated groups showed increased expression of IGF-1R, p-AKT, and p-Foxo3a, which are involved in muscle generation.

Furthermore, for MuRF1, a key gene associated with muscle atrophy, its expression, which had been significantly elevated due to CCM treatment, was reduced in both apigenin-treated and apigenin-glycated albumin-treated groups. Notably, the MuRF1 expression level in the apigenin-glycated albumin-treated group was significantly lower than in the group treated with apigenin alone, confirming that the apigenin-glycated albumin exhibited a markedly enhanced synergistic effect compared to apigenin alone.

### Experimental Example 6: Verification of the Effect of Inhibiting Muscle Cell Atrophy Induced by Anticancer Drugs

The improvement effect of apigenin-glycated albumin on muscle atrophy that may be caused by administration of anticancer drugs was verified.

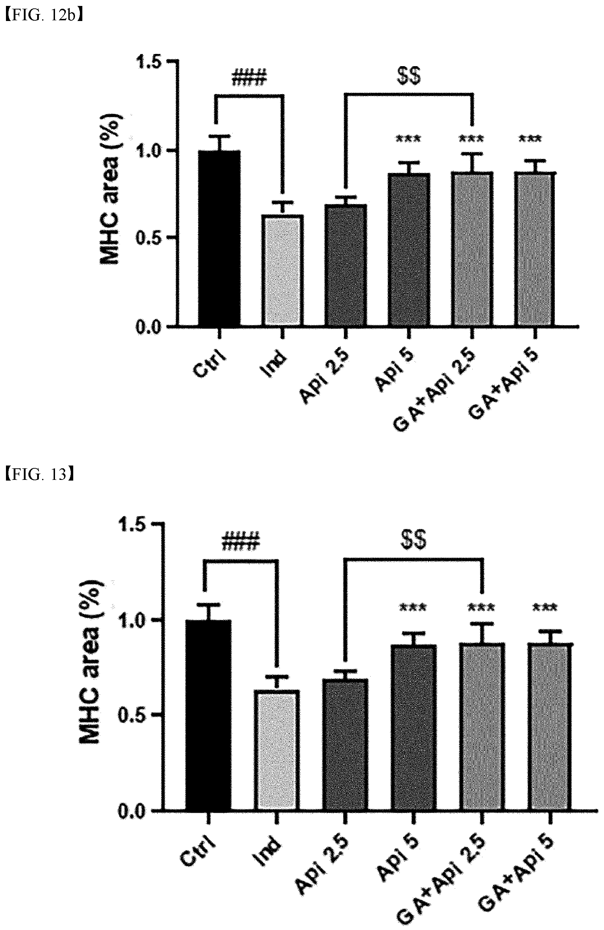

First, mouse muscle cells (C2C12) were cultured, and 40 µM of cisplatin was added along with apigenin alone or apigenin-glycated albumin at concentrations of 2.5 and 5 µM, followed by incubation for 48 hours. Then, immunofluorescence staining was performed to assess the level of differentiation into myotubes, as shown in FIGS. 12a and 12b.

As shown in FIGs. 12a and 12b, although muscle cell atrophy was induced by cisplatin, in the cell groups treated with apigenin or apigenin-glycated albumin, the atrophy caused by cisplatin was inhibited. In particular, the cell group treated with apigenin-glycated albumin at a concentration of 2.5 µM exhibited a more significant atrophy-inhibiting effect than the cell group treated with the same concentration of apigenin alone.

Additionally, proteins were extracted from each experimental cell group, and western blot analysis was conducted to examine the expression level of MyoG (myogenin), which induces muscle generation. The results are shown in FIG. 13. Referring to FIG. 13, MHC and MyoG expression, which had been reduced by cisplatin treatment, were increased by treatment with apigenin. Notably, in the cell group treated with apigenin-glycated albumin, the expression level of MyoG, which induces muscle generation, was significantly increased.

These results confirm that apigenin and glycated albumin exhibit a synergistic effect in improving muscle atrophy induced by anticancer drug administration.

### Experimental Example 7: Verification of In Vivo Targeting Ability of Glycated Albumin

Using the albumin nanocomposite of the present disclosure that has GLUT-targeting capability, the possibility of formulation as an injectable preparation via actual administration routes was confirmed.

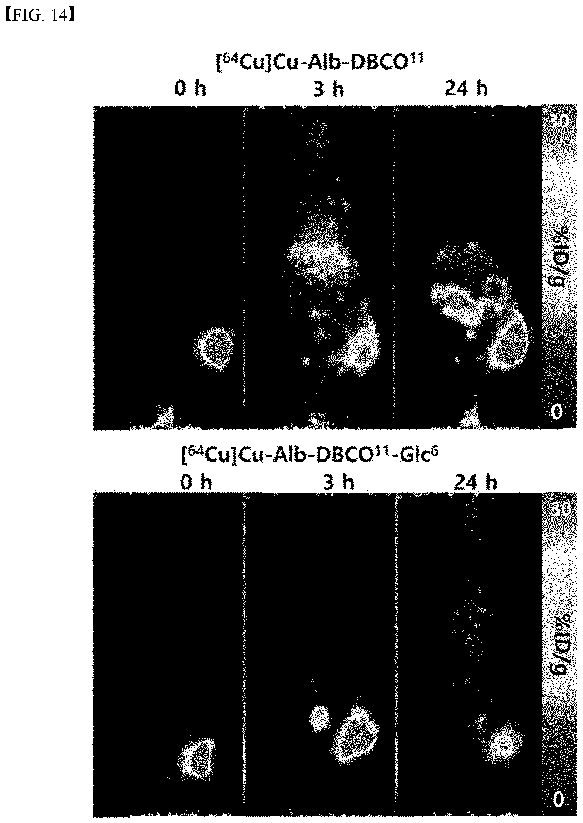

To evaluate how long the albumin nanocomposite remains at the injection site and delivers the drug to the diseased region, an intramuscular injection experiment was conducted, and the results are shown in FIG. 14.

Referring to FIG. 14, the albumin platform (Albumin(11)) introduced with 11 ADIBO functional groups, and the glycated albumin nanocomposite [Glc(6)-Albumin(11), i.e., albumin with 11 ADIBO groups, 6 of which were substituted with glucose], were intramuscularly injected. Nuclear imaging using radioisotopes was performed at 0, 3, and 24 hours after injection, and very high retention at the injection site was observed at all time points.

This result indicates that, unlike conventional drugs that are rapidly absorbed after intramuscular injection and thus fail to sufficiently accumulate in the diseased region, the present technology enables optimal pharmacological concentration to be achieved with a smaller amount of drug, thereby solving the conventional limitations and providing a significant improvement.

### Experimental Example 8: Evaluation of the Simultaneous Distribution of Glycated Albumin and Apigenin

To visualize and analyze the dynamics of the drug and albumin platform in real time after simultaneous injection into mice, different radioisotopes were labeled to albumin and the drug, respectively.

The labeling method using the SPECT isotope I-125 was established for apigenin (¹²⁵ I-Api), and the PET isotope Cu-64 was labeled to albumin using click chemistry (⁶⁴Cu-Alb). In addition, to verify the targeting ability conferred by glycation, the PET isotope Cu-64 was also labeled to glucose-introduced albumin (⁶⁴Cu-Glc-Alb).

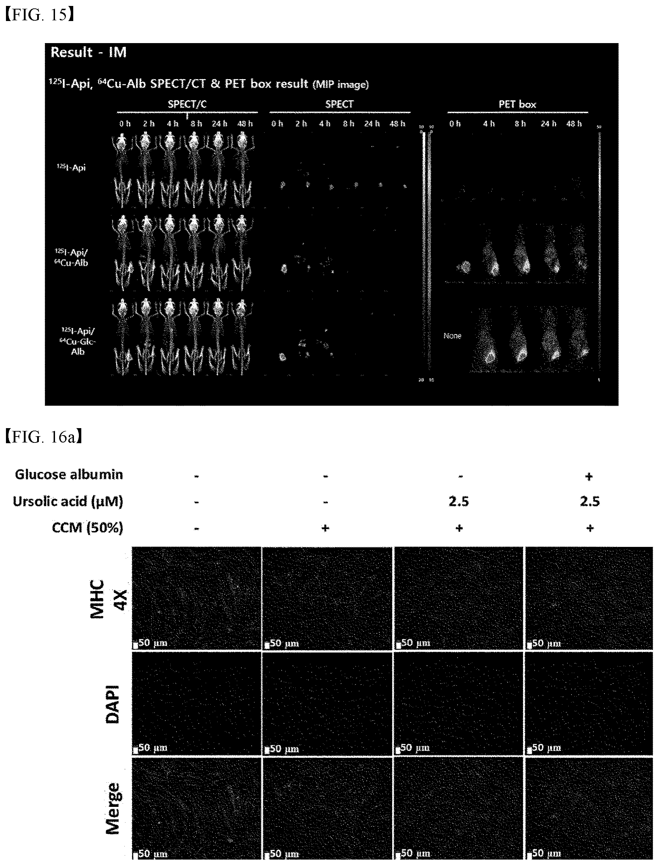

Labeled apigenin (1251-Api), the composite of apigenin and albumin (125I-Api/64Cu-Alb), and the composite of apigenin and glycated albumin (125I-Api/64Cu-Glc-Alb) were intramuscularly injected, and SPECT and PET images were sequentially acquired, as shown in FIG. 15.

Referring to FIG. 15, in mice injected with labeled apigenin (1251-Api), the apigenin remained at the injection site, and uniform imaging signals indicated that no absorption, metabolism, or excretion occurred.

Meanwhile, in the cases of the composite of apigenin and albumin (125I-Api/64Cu-Alb) and the composite of apigenin and glycated albumin (125I-Api/64Cu-Glc-Alb), re-circulation through the lymphatic system into the bloodstream followed by intestinal excretion was observed after intramuscular injection.

This suggests that the composite of apigenin and albumin is absorbed, metabolized, and excreted, indicating enhanced drug absorption and retention at specific sites to exhibit pharmacological efficacy.

Furthermore, since the PET signal pattern remained the same up to 8 hours, it was confirmed that the drug exists in composite form until absorbed, and is then separated upon metabolism and excretion, showing potential for targeted therapy to specific cells.

### Preparation Example 2: Preparation of Ursolic Acid-Glycated Albumin Composite

Ursolic acid was loaded onto glycated albumin prepared by the method of Preparation Example 1-1.

Ursolic acid at 10 equivalents based on albumin was mixed with albumin, reacted for 1.5 hours, and purified using a filter to obtain the ursolic acid-glycated albumin composite.

### Experimental Example 9: Evaluation of the Improving Effect of Ursolic Acid-Glycated Albumin on Muscle Atrophy

An experiment was conducted to evaluate the effect of the ursolic acid-glycated albumin on improving muscle atrophy.

Mouse muscle cells (C2C12) were first cultured, and then their medium was replaced with cancer cell-conditioned medium (CCM) from cultured mouse colon carcinoma cells (CT26) to induce muscle atrophy. The cells were treated with either 2.5 µM of ursolic acid alone or ursolic acid-glycated albumin and incubated for 48 hours. Thereafter, fluorescence expression was confirmed to evaluate the level of myotube differentiation.

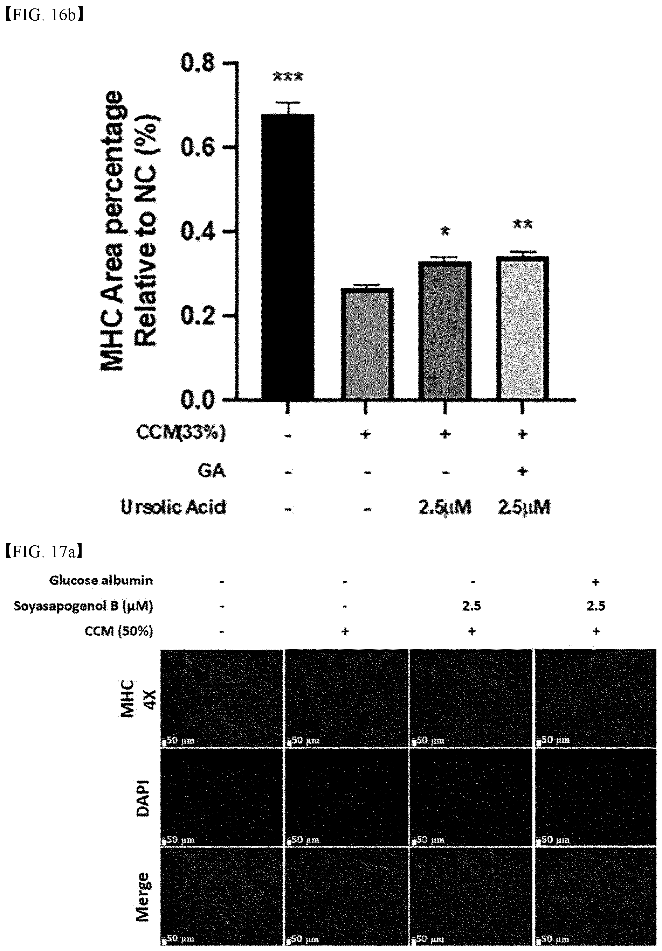

As shown in FIGs. 16a and 16b, MHC expression was increased in both groups treated with ursolic acid alone and the ursolic acid-glycated albumin in the CCM-induced atrophied cells, indicating that the muscle cells improved.

In particular, compared to ursolic acid alone, MHC expression was significantly higher in the group treated with ursolic acid-glycated albumin, demonstrating that the ursolic acid-glycated albumin exhibits a synergistic effect superior to that of ursolic acid alone.

### Preparation Example 3: Preparation of Soyasapogenol B-Glycated Albumin Composite

Soyasapogenol B at 10 equivalents based on glycated albumin prepared by the method of Preparation Example 1-1 was mixed with albumin, reacted for 1.5 hours, and purified using a filter to obtain the soyasapogenol B-glycated albumin composite.

### Experimental Example 10: Evaluation of the Improving Effect of Soyasapogenol B-Glycated Albumin on Muscle Atrophy

An experiment was conducted to evaluate the effect of soyasapogenol B-glycated albumin on improving muscle atrophy.

Mouse muscle cells (C2C12) were first cultured, and their medium was replaced with cancer cell-conditioned medium (CCM) from cultured mouse colon carcinoma cells (CT26) to induce muscle atrophy. The cells were treated with either 2.5 µM of soyasapogenol B alone or soyasapogenol B-glycated albumin and incubated for 48 hours. Thereafter, fluorescence expression was confirmed to evaluate the level of myotube differentiation.

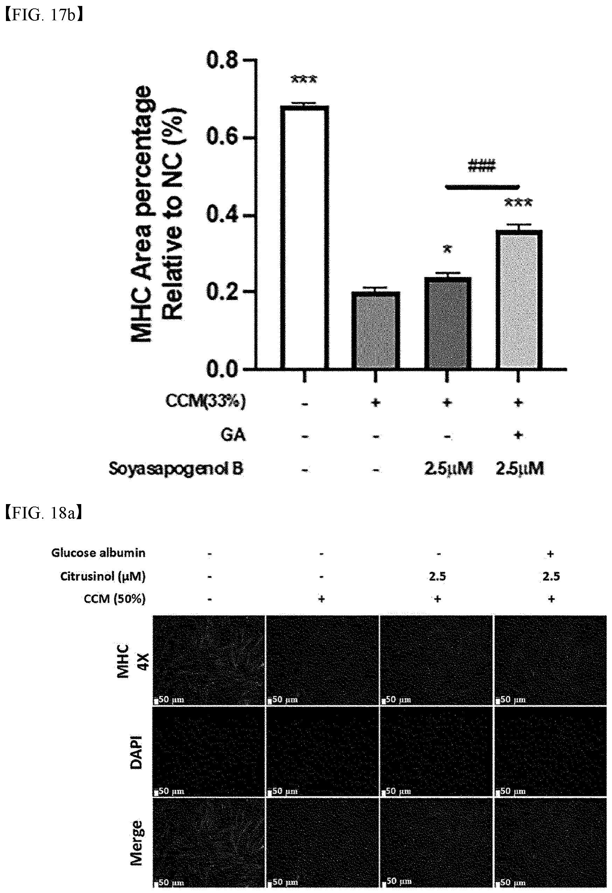

As shown in FIGs. 17a and 17b, MHC expression was increased in both groups treated with soyasapogenol B alone and soyasapogenol B-glycated albumin in the CCM-induced atrophied cells, indicating muscle cell improvement.

In particular, MHC expression was significantly higher in the group treated with soyasapogenol B-glycated albumin than in the group treated with soyasapogenol B alone, confirming that the soyasapogenol B-glycated albumin exhibits a superior synergistic effect compared to soyasapogenol B alone.

### Preparation Example 4: Preparation of Citrusinol-Glycated Albumin Composite

Citrusinol at 10 equivalents based on glycated albumin prepared by the method of Preparation Example 1-1 was mixed with albumin, reacted for 1.5 hours, and purified using a filter to obtain the citrusinol-glycated albumin composite.

### Experimental Example 11: Evaluation of the Improving Effect of Citrusinol-Glycated Albumin on Muscle Atrophy

An experiment was conducted to evaluate the effect of citrusinol-glycated albumin on improving muscle atrophy.

Mouse muscle cells (C2C12) were first cultured, and their medium was replaced with cancer cell-conditioned medium (CCM) from cultured mouse colon carcinoma cells (CT26) to induce muscle atrophy. The cells were treated with either 2.5 µM of citrusinol alone or citrusinol-glycated albumin and incubated for 48 hours. Thereafter, fluorescence expression was confirmed to evaluate the level of myotube differentiation.

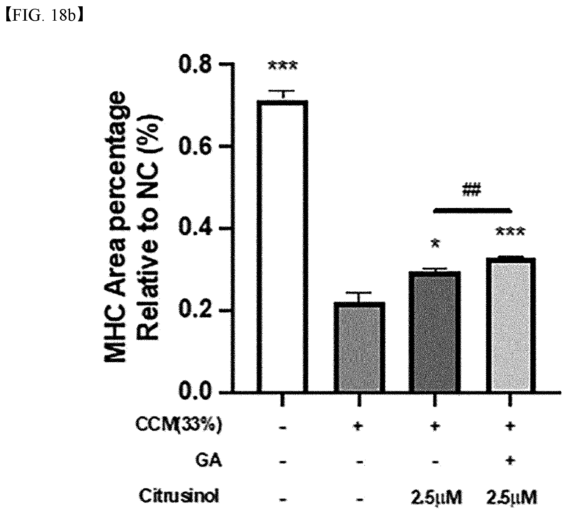

As shown in FIGs. 18a and 18b, MHC expression was increased in both groups treated with citrusinol alone and citrusinol-glycated albumin in the CCM-induced atrophied cells, indicating muscle cell improvement.

In particular, MHC expression was significantly higher in the group treated with citrusinol-glycated albumin than in the group treated with citrusinol alone, confirming that the citrusinol-glycated albumin exhibits a synergistic effect superior to that of citrusinol alone.

The above description has been provided for some embodiments of the present disclosure, but the present disclosure is not limited to such embodiments. It may be modified and altered in various ways without departing from the spirit of the present disclosure, and such modifications and alterations should also be construed as falling within the technical scope of the present disclosure.

## Claims

1. An albumin nanocomposite obtained by a click chemistry reaction between albumin conjugated with an azide (N₃) or cyclooctyne functional group and a transmitter conjugated with an azide (N₃) or cyclooctyne functional group,
wherein a phytochemical is loaded on the surface of the albumin,
wherein the transmitter comprises a glucosyl group, and
when the albumin is conjugated with the azide functional group, the transmitter is conjugated with the cyclooctyne functional group, and when the albumin is conjugated with the cyclooctyne functional group, the transmitter is conjugated with the azide functional group.

2. The albumin nanocomposite of claim 1,
wherein the phytochemical is at least one selected from the group consisting of a phenolic compound and a triterpene.

3. The albumin nanocomposite of claim 2,
wherein the phenolic compound is at least one selected from the group consisting of apigenin, citrusinol, flavone, flavonol, flavanone, flavanol, isoflavone, anthocyanin, stilbenoid, caffeic acid, p-coumaric acid, chrysin, tectochrysin, primetin, acacetin, luteolin, tangeritin, quercetin, kaempferol, genistein, daidzein, naringenin, hesperetin, cyanidin, catechin, curcumin, epigallocatechin gallate and resveratrol.

4. The albumin nanocomposite of claim 2,
wherein the triterpene is at least one selected from the group consisting of ursolic acid, soyasapogenol A, soyasapogenol B, ursane, oleanane, lupane, saponin, sterol, oleanolic acid, lupeol, corosolic acid, maslinic acid, betulinic acid, ginsenoside, beta-sitosterol, campesterol, stigmasterol, lanosterol and germanicol.

5. The albumin nanocomposite of claim 1,
wherein the azide or cyclooctyne functional group introduced into the albumin ranges from 1 to 14.

6. The albumin nanocomposite of claim 1,
wherein the nanocomposite comprises 4 to 8 glucosyl groups.

7. The albumin nanocomposite of claim 1,
wherein the azide or cyclooctyne functional group is conjugated to the 6th carbon of the glucosyl group.

8. The albumin nanocomposite of claim 1,
wherein the phytochemical is loaded in an amount of 1 to 20 molecules.

9. The albumin nanocomposite of claim 1,
wherein the transmitter further comprises a radioactive isotope, and the radioactive isotope is at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹Fe, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I , ¹²⁵I, ¹²⁴I, ¹²³I, ¹⁸⁶Re, ¹⁸⁸Re, ²²⁵Ac, ²¹²Pb, ^{117m}Sn and ¹⁷⁷Lu.

10. The albumin nanocomposite of claim 9,
wherein the radioactive isotope is labeled by a chelating agent, and the chelating agent is at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N2S2 (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino]-6,11,17,22-tetraazaheptaeicosane]thiourea) and HYNIC (hydrazinonicotinic acid).

11. The albumin nanocomposite of claim 1,
wherein the transmitter further comprises a fluorescent material, and the fluorescent material is at least one selected from the group consisting of FNR (Ferredoxin NADP(+) reductase), a cyanine-based fluorescent material, TAMRA (tetramethylrhodamine-5-maleimide), Flamma^{®} fluorescent material and ICG (indocyanine green).

12. The albumin nanocomposite of claim 1,
wherein the nanocomposite selectively targets a macrophage with overexpressed GLUT (Glucose Transporter) to deliver the phytochemical.

13. A pharmaceutical composition for preventing or treating a muscle disease, comprising the albumin nanocomposite according to any one of claims 1 to 12.

14. The pharmaceutical composition of claim 13,
wherein the muscle disease is induced by an anticancer agent or an inflammatory response.

15. The pharmaceutical composition of claim 14,
wherein the anticancer agent is at least one selected from the group consisting of etoposide, adriamycin, vincristine, cyclophosphamide, ifosfamide, cisplatin, vinorelbine, paclitaxel, docetaxel, gemcitabine and pemetrexed.

16. The pharmaceutical composition of claim 13,
wherein the muscle disease is selected from the group consisting of sarcopenia and muscular atrophy.

17. The pharmaceutical composition of claim 13,
wherein the pharmaceutical composition suppresses muscle loss and promotes the proliferation of myoblasts and differentiation into muscle cells.

18. A food composition for preventing or improving a muscle disease, comprising the albumin nanocomposite according to any one of claims 1 to 12.
